# EUROPEAN PATENT APPLICATION

(11) **EP 2 997 884 A1**
(43) Date of publication of application: **23.03.2016**
(21) Application number: 15000059.4
(22) Date of filing: 13.01.2015
(51) Int. Cl.: A61B 5/00, D03D 1/00

(54) **Combination method of multiple-layer conductive physiological detection structure**

(30) Priority: 16.09.2014 CN 201413131855
(71) Applicant: King's Metal Fiber Technologies Co., Ltd., Taichung City (TW)
(72) Inventor: Kang, Yu-Hsun, Taipei City (TW); Wang, Hao-Chen, Taipei City (TW); Liao, Shu-Fen, Taipei City (TW)
(74) Representative: Kador & Partner

(57) **Abstract**

The present invention relates to a combination method of a multiple-layer conductive physiological detection structure, which includes conductive fabric (100) in the form of a patch and a conductive woven band (200) in the form of a strip-like band. The method includes: combining the conductive fabric (100) and the conductive woven band (200) together as a unitary device through a fusion operation. The fusion operation includes high frequency fusion or ultrasonic fusion.

## Description

### FIELD OF THE INVENTION

The present invention relates to a combination method applicable to a structure for detecting a physiological signal, and in particular to a multiple-layer conductive physiological detection structure that is applicable as a conductive structure for physiological detection and formed of connection of multiple layers and which is a formed through radio frequency, high frequency, or ultrasonic fusion.

### BACKGROUND OF THE INVENTION

In the known techniques, when a physiological detection device is applied to human physiological detection, the detection must be made at a fixed site or a physiological detection device that is made through multiple operations of sewing or bonding is set in contact with a human skin. It is generally impossible to allow the physiological detection device to be carried by a person in a comfortable manner for timely performance of physiological detection. Further, the conventional physiological detection device must be subjected to multiple times of processing, either sewing or bonding, during the manufacture thereof. This is not only a waste of money but also consuming of time and labor and it often cannot achieve desired stability.

The conventional structure has complicated connection and when a user is making a movement, it is often that the detected physiological signal is cut off. When the user's motion makes stretching or elongation, it may even cause shorting or opening of the device connected with the physiological detection signals. There is no physiological signal detection device or structure available that has good performance and is satisfactory.

### SUMMARY OF THE INVENTION

An object of the present invention is to provide a combination method of a multiple-layer conductive physiological detection structure, wherein a conductive that is applicable to physiological detection and formed of connection of multiple layers is combined through a single fusion operation of radio frequency, high frequency, or ultrasonic fusion so as to achieve various advantages of saving cost and improving electrical conductivity.

According to an embodiment of the present invention, a combination method of a multiple-layer conductive physiological detection structure is disclosed, which comprises at least one piece of conductive fabric and a conductive woven band. The conductive woven band and the conductive fabric are combined together through a fusion operation.

In an embodiment of the present invention, the conductive fabric is formed by weaving electrically conductive units and yarns together.

In an embodiment of the present invention, a piece of three-dimensional mixed-yarn conductive fabric is further included. The three-dimensional mixed-yarn conductive fabric is formed by weaving a mixture of conductive yarns, support yarns, and weaving yarns. The three-dimensional mixed-yarn conductive fabric and the conductive fabric make use of the conductive woven band to have the three combined together. The three-dimensional mixed-yarn conductive fabric is of a structure having a protruding configuration.

In an embodiment of the present invention, the conductive fabric, the combination of the conductive woven band, or the three-dimensional mixed-yarn conductive fabric is achieved through bonding, fusion, or welding to form a multiple-layer physiological detection structure.

In an embodiment of the present invention, at least two pieces of conductive fabric are included and the conductive woven band is positioned between the pieces of conductive fabric to be combined therewith and connected thereto.

In an embodiment of the present invention, the three-dimensional mixed-yarn conductive fabric further comprises a protrusion member contained therein. In a practical application, the protrusion member is of a structure having a protruding configuration or the protrusion member comprises foam, silicon rubber, or cotton.

In an embodiment of the present invention, the yarns are formed of chemical fibers, natural fibers, or mix spinning of chemical fibers and natural fibers.

In an embodiment of the present invention, the conductive fabric comprises stainless steel nonwoven fabric.

In an embodiment of the present invention, the conductive woven band is of a structure formed according to a band weaving style.

In an embodiment of the present invention, the conductive woven band comprises an electrically conductive unit, which is wrapped around or extends across the conductive woven band in a nonlinear manner.

In an embodiment of the present invention, the electrically conductive unit comprises a conductive yarn or a conductive wire.

In an embodiment of the present invention, the conductive woven band further comprises at least two elastic yarns and the elastic yarns are arranged at two lateral sides of the conductive woven band.

In an embodiment of the present invention, the fusion operation comprises ultrasonic fusion or high frequency fusion.

In an embodiment of the present invention, a piece of three-dimensional mixed-yarn conductive fabric that is formed by weaving a mixture of conductive yarns, support yarns, and weaving yarns is further included, wherein the conductive fabric, the conductive woven band, and the three-dimensional mixed-yarn conductive fabric are fused together as a unitary device through ultrasonic fusion.

In an embodiment of the present invention, a time period in which the fusion operation is performed is adjusted and determined according to an amount of plastics contained in the conductive fabric or the conductive woven band.

In an embodiment of the present invention, the conductive fabric is formed by weaving electrically conductive units and yarns together..

In an embodiment of the present invention, the conductive fabric comprises stainless steel nonwoven fabric.

In an embodiment of the present invention, the conductive woven band comprises an elastic conductive woven band and the elastic conductive woven band is composed of yarns of elasticity and electrically conductive units.

In an embodiment of the present invention, the conductive woven band is a non-elastic conductive woven band and the non-elastic conductive woven band is composed of inelastic yarns and electrically conductive units.

The multiple-layer conductive physiological detection structure according to an embodiment of the present invention is a multiple-layer conductive structure that applies ultrasonic, radio frequency, or high frequency fusion to fuse the conductive fabric, the conductive woven band, or the three-dimensional mix-yarn conductive fabric together to be applicable, in a portable manner, to detection of physiological signals so as to achieve various purposes of being portable, saving cost, connection of multiple layers, and stability.

### BRIEF DESCRIPTION OF THE DRAWINGS

The present invention will be apparent to those skilled in the art by reading the following description of preferred embodiments thereof with reference to the drawings, in which:
Figure 1 is an exploded view showing a multiple-layer conductive physiological detection structure in accordance with the present invention;
Figure 2 is a perspective view showing the multiple-layer conductive physiological detection structure in accordance with the present invention;
Figure 3A is a schematic view showing a conductive woven band of the multiple-layer conductive physiological detection structure in accordance with the present invention;
Figure 3B is a partial enlarged view of the conductive woven band of the multiple-layer conductive physiological detection structure in accordance with the present invention;
Figure 4 is an exploded view showing an embodiment of the multiple-layer conductive physiological detection structure in accordance with the present invention;
Figure 5A is a perspective view showing the embodiment of the multiple-layer conductive physiological detection structure in accordance with the present invention;
Figure 5B is a cross-sectional view showing the embodiment of the multiple-layer conductive physiological detection structure in accordance with the present invention; and
Figure 6 is a perspective view showing another embodiment of the multiple-layer conductive physiological detection structure in accordance with the present invention.

### DETAILED DESCRIPTION OF THE PREFERRED EMBODIMENTS

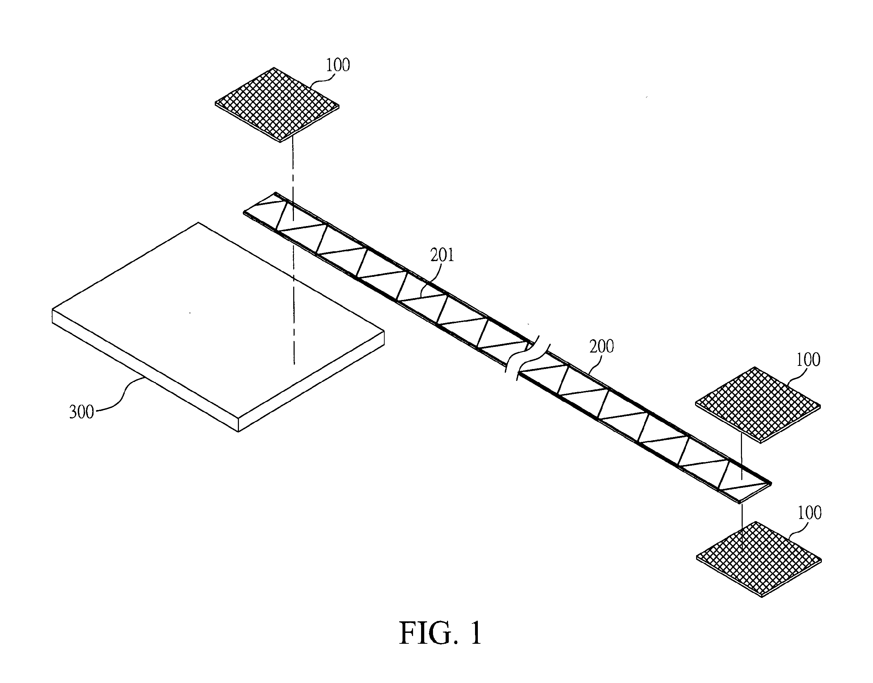

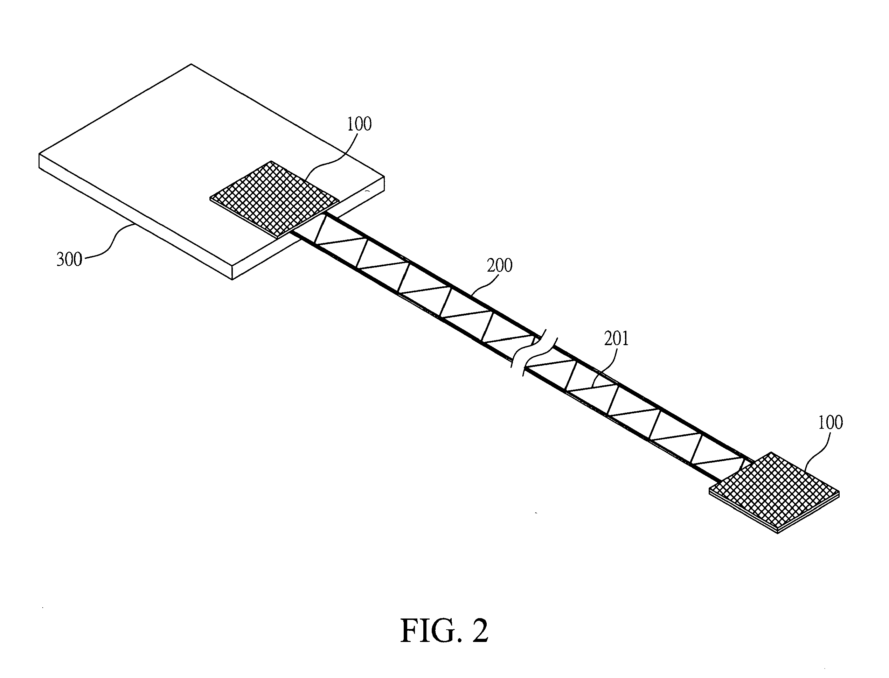

With reference to the drawings and in particular to Figures 1 and 2, an exploded view and a perspective view are given to illustrate a multiple-layer conductive physiological detection structure according to the present invention. As shown in Figures 1 and 2, the multiple-layer conductive physiological detection structure according to the present invention comprises at least a piece of conductive fabric 100 and a conductive woven band 200. The conductive fabric 100 can be a patch and the conductive woven band 200 can be a strip-like band, which is formed by combining yarns of elasticity and electrically conductive units. Through application of ultrasonic, radio frequency, or high frequency fusion, the conductive fabric 100 and the conductive woven band 200 are fused together as a unitary device and, in addition, it is possible to place a conductive woven band 200 between two pieces of the conductive fabric 100 and are fused together as a unitary device by application of ultrasonic, radio frequency, or high frequency fusion.

It is also noted here that the instant embodiment may further comprise a three-dimensional mixed-yarn conductive fabric 300, which is formed by weaving a mixture of yarns including conductive yarns, support yarns, and weaving yarns and shows a structure of a predetermined thickness through the use of the support yarns and specific weaving styles. Further, the three-dimensional mixed-yarn conductive fabric 300 and the conductive fabric 100 make use of the conductive woven band 200 to have the three combined together. Next, the conductive woven band 200 is placed between the conductive fabric 100 and the three-dimensional mixed-yarn conductive fabric 300 and are fused together as a unitary device through application of ultrasonic, radio frequency, or high frequency fusion. The three-dimensional mixed-yarn conductive fabric can be, practically, of a structure having a protruding configuration and can be designed as a protruding configuration through proper styles of weaving.

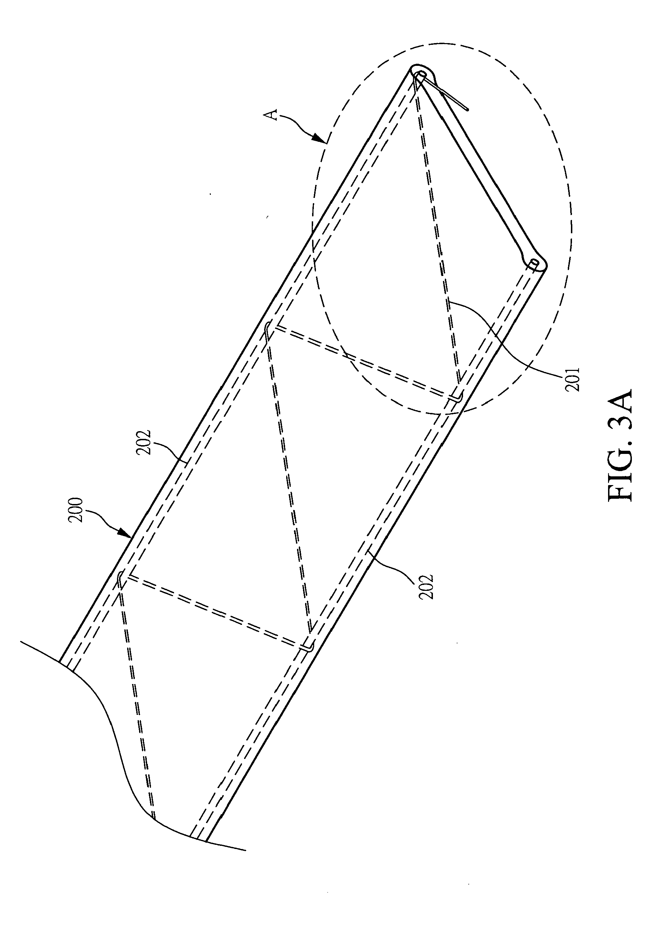

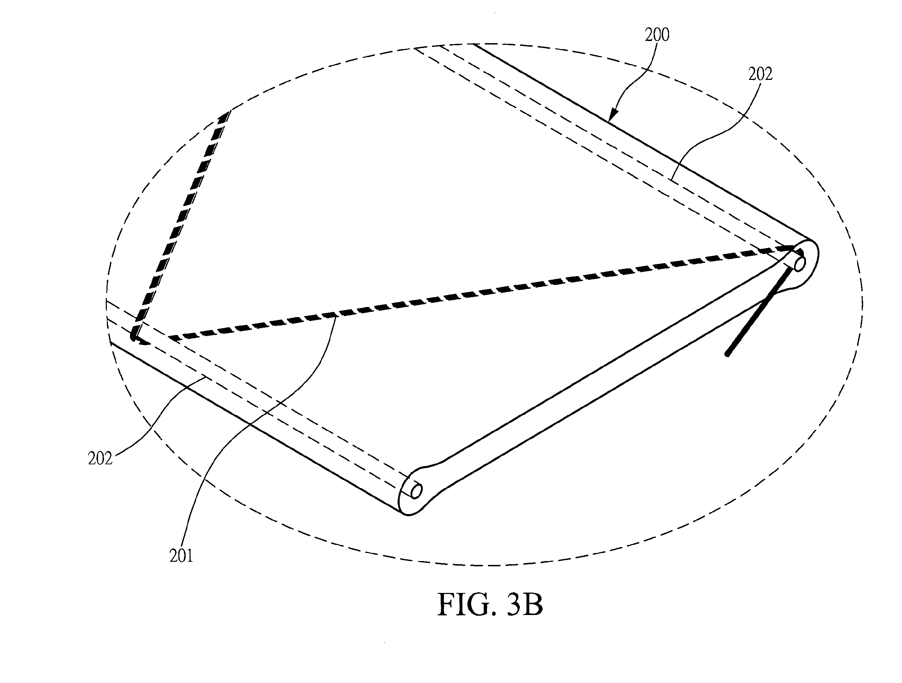

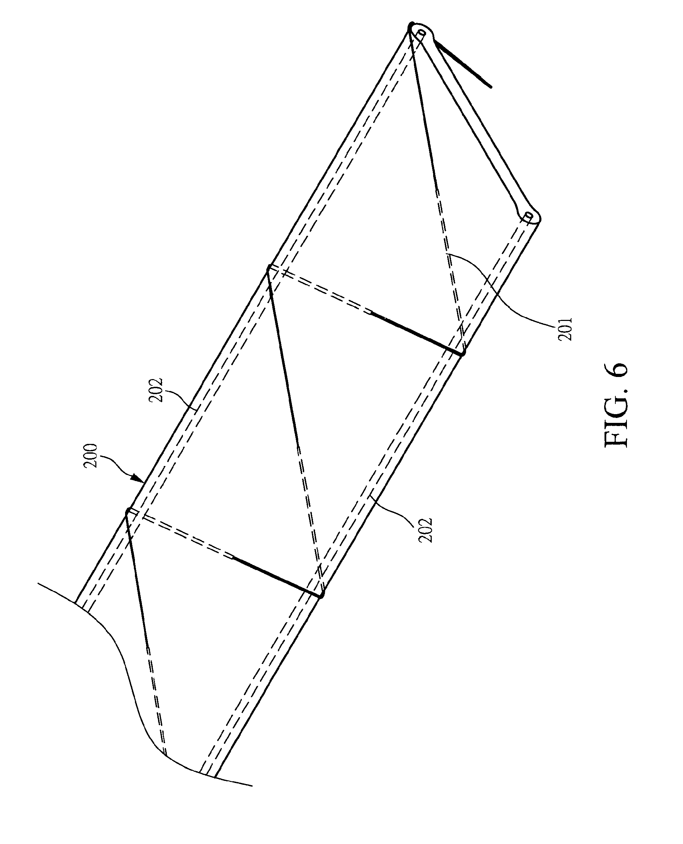

Referring to Figures 3A and 3B, schematic views are given to illustrate the conductive woven band of the multiple-layer conductive physiological detection structure according to the present invention. As shown in Figures 3A and 3B, the electrically conductive unit 201 is arranged on a surface of the conductive woven band 200 in a nonlinear form. Further, the electrically conductive units 201 can be composed of conductive yarns or conductive wires. In a practical application, the electrically conductive units 201 may be alternatively set on opposite sides of the conductive woven band 200. The conductive woven band 200 can be of a structure formed through an elastic weaving process or the electrically conductive units 201 of the conductive woven band 200 are arranged to wrap the conductive woven band 200 in a nonlinear manner. Further, as shown in Figure 6, the electrically conductive unit 201 of the conductive woven band 200 may be arranged to alternately extend across the top and bottom of the conductive woven band 200.

Further, as shown in Figures 3A and 3B, the multiple-layer conductive physiological detection structure of the present invention may further comprises elastic yarns 202. In a practical application, the elastic yarns 202 are arranged at two lateral sides of the conductive woven band 200. The use of the two of the electrically conductive units 201 and the elastic yarns 202 in the conductive woven band 200 is not limited to what illustrated in the drawing, which generally illustrates the conductive woven band 200 can be made up of the two of the electrically conductive units 201 and the elastic yarns 202 and the combining process and the weaving style thereof can be extended according to the design of an actual product. The elastic yarns 202 help improve the tensile strength. The arrangement of the electrically conductive units 201 in the conductive woven band 200 can be of a Z-shaped configuration, which provides the electrically conductive units 201 with an effect of cushioning and elongation when the conductive woven band 200 is elongated. Further, with the arrangement of the elastic yarns 202, elastic restoration rate of the elastic conductive woven band 200 can be improved.

In practice, the electrically conductive units are used in the conductive woven band such that with the elastic extendibility of the conductive woven band and physiological signal transmission through the electrically conductive unit, when a user makes a movement and stretches the body thereof, the conductive woven band provides a certain level of elasticity to protect the electrically conductive unit for transmitting physiological detection signals so that the electrically conductive units can woven in and in combination with the conductive woven band and can also allow for various different designs according to actual needs of products and are not limited to the embodiment.

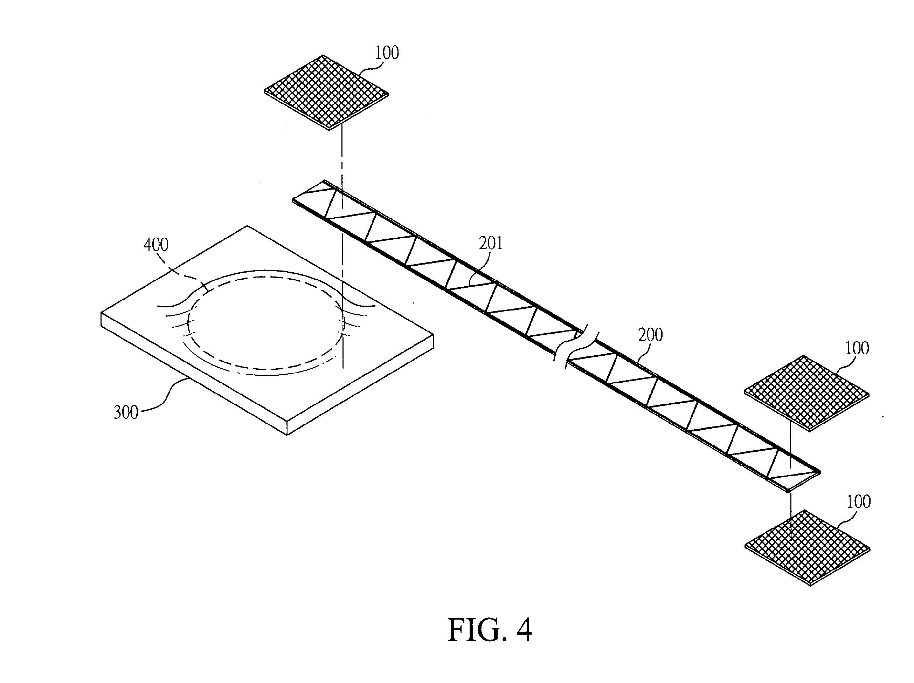

Referring to Figures 4-5B, an embodiment of the multiple-layer conductive physiological detection structure according to the present invention is shown. As shown in Figures 4-5B, the three-dimensional mixed-yarn conductive fabric 300 may further comprise therein a protrusion member 400. The protrusion member 400 can be made of a material of foam, silicon rubber, and cotton. In the application of ultrasonic, radio frequency, or high frequency fusion, the protrusion member 400 of the three-dimensional mixed-yarn conductive fabric 300 shows a predetermined thickness that raised beyond a surface of the multiple-layer conductive physiological detection structure of the present invention in the entirety thereof so as to enhance the contact engagement between the surface area and human skin and to prevent discomfort of the human body caused by the fused material of the conductive fabric.

Further, the conductive fabric 100, in a practical application, can be a piece of stainless steel nonwoven fabric. In another embodiment, the yarns used in the present invention, in a practical application, can be formed of chemical fibers, natural fibers, or mix spinning of chemical fibers and natural fibers. The electrically conductive units and the yarns are mixed and woven with a ratio therebetween adjusted and determined by practical needs.

A combination method of the multiple-layer conductive physiological detection structure according to the present invention comprises conductive fabric and a conductive woven band. The conductive fabric can be a patch and is a piece of conductive fabric that is formed by mixing and weaving together electrically conductive units and yarns. The conductive woven band can be a strip-like band. The method for combining the conductive fabric and the conductive woven band is application of radio frequency, high frequency, or ultrasonic fusion to have them combined as a unitary device. Further, the electrically conductive units can be conductive yarns or conductive wires.

In a practical application, the combination method of the multiple-layer conductive physiological detection structure according to the present invention is to combine multiple layers of conductive structure or fabric together as a unity through application of the above-mentioned radio frequency, high frequency, or ultrasonic fusion so that the multiple-layer conductive physiological detection structure can be integrally and efficiently combined as a unitary device to thereby shorten the manufacture time and reduce the manufacture cost.

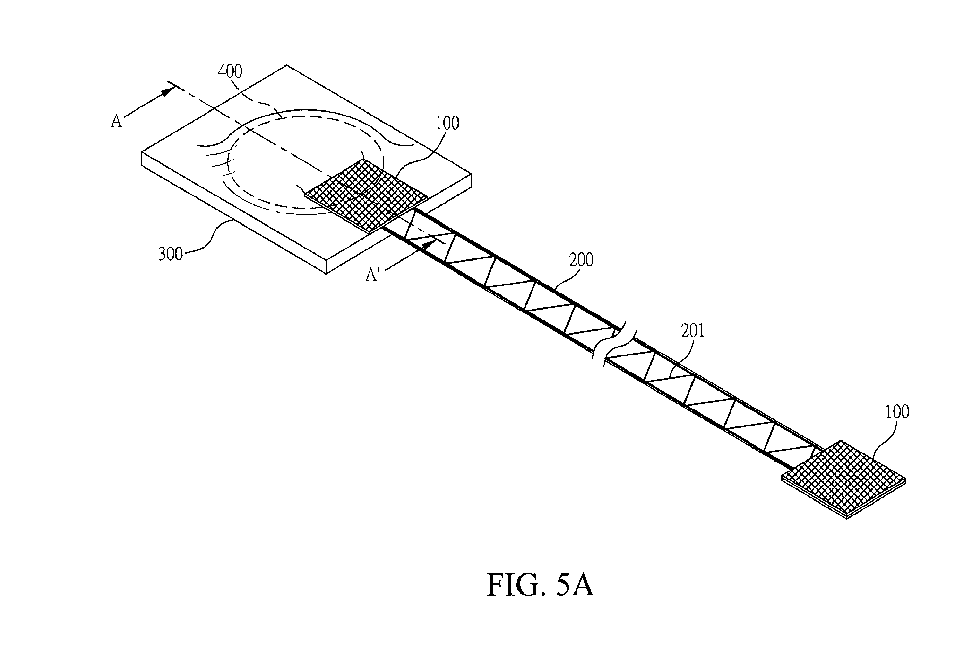

Further, as shown in Figures 4-5B, a three-dimensional mixed-yarn conductive fabric 300 that is formed by weaving a mixture of yarns of conductive yarns, support yarns, and weaving yarns. The three of the conductive fabric 100, the conductive woven band 200, and the three-dimensional mixed-yarn conductive fabric 300 are fused together as a unitary device through ultrasonic fusion. With the three-dimensional mixed-yarn conductive fabric 300 that has a predetermined thickness, the surface area in contact with human skin and stability can be increased and, also, the conductivity and stability for receiving physiological signals are enhanced.

Further, in the combination method of the multiple-layer conductive physiological detection structure according to the present invention, the time period for the application of the radio frequency, high frequency, or ultrasonic fusion is adjusted and determined according to the amount of plastics contained in the conductive fabric or the conductive woven band. Further, the conductive woven band is formed by combining yarns and electrically conductive units, in which the yarns can be of an elastic or non-elastic structure.

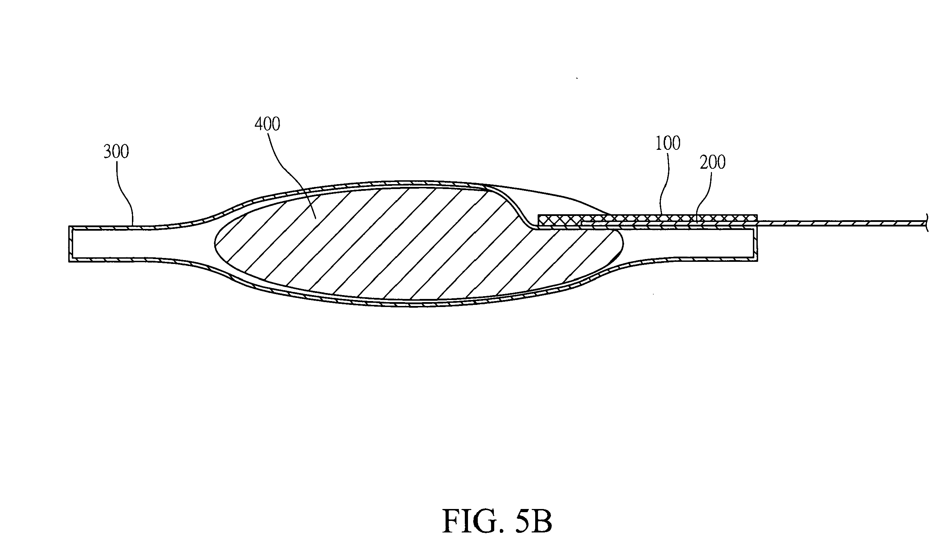

As shown in Figure 5B, the application of a radio frequency, high frequency, or ultrasonic fusion operation integrally forms the multiple-layer conductive physiological detection structure, where connection among the three is made, through fusion and pressing bonding, so that the surface thereof is lower than the surface of the three-dimensional mixed-yarn conductive fabric 300 that is in contact engagement with the human skin to prevent causing discomfort of the human skin. Further, the three-dimensional mixed-yarn conductive fabric 300 may additionally include therein a protrusion member 400, or weaving styles can be selected to make the three-dimensional mixed-yarn conductive fabric 300 in the form of a projection to increase the contact surface area with human skin.

In summary, the present invention provides a multiple-layer conductive physiological detection structure, which is integrally formed as a unitary device through application of radio frequency, high frequency, or ultrasonic fusion so as to shorten the manufacture time and save manufacture cost and improve stability and comfortableness of the multiple-layer conductive physiological detection structure, providing an effect of multiple functions.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A combination method of a multiple-layer conductive physiological detection structure, comprising conductive fabric (100) in the form of a patch and a conductive woven band (200) in the form of a strip-like band, the method comprising: combining the conductive fabric (100) and the conductive woven band (200) together as a unitary device through a fusion operation.

2. The combination method of the multiple-layer conductive physiological detection structure as claimed in Claim 1, wherein the fusion operation comprises ultrasonic fusion or high frequency fusion.

3. The combination method of the multiple-layer conductive physiological detection structure as claimed in Claim 1 or 2 further comprising weaving a mixture of conductive yarns, support yarns, and weaving yarns to form three-dimensional mixed-yarn conductive fabric (300), combining the conductive fabric (100), the conductive woven band (200), and the three-dimensional mixed-yarn conductive fabric (300) together as a unitary device through ultrasonic fusion.

4. The combination method of the multiple-layer conductive physiological detection structure as claimed in any of the preceding claims, wherein a time period in which the fusion operation is performed is adjusted and determined according to an amount of plastics contained in the conductive fabric (100) or the conductive woven band (200).

5. The combination method of the multiple-layer conductive physiological detection structure as claimed in any of the preceding claims, wherein the conductive fabric (100) is formed by weaving electrically conductive units (201) and yarns together.

6. The combination method of the multiple-layer conductive physiological detection structure as claimed in any of the preceding claims, wherein the conductive fabric (100) comprises stainless steel nonwoven fabric.

7. The combination method of the multiple-layer conductive physiological detection structure as claimed in any of the preceding claims, wherein the conductive woven band (200) comprises an elastic conductive woven band, the elastic conductive woven band being composed of yarns of elasticity and electrically conductive units (201).

8. The combination method of the multiple-layer conductive physiological detection structure as claimed in any of the preceding claims, wherein the conductive woven band (200) is a non-elastic conductive woven band, the non-elastic conductive woven band being composed of inelastic yarns and electrically conductive units (201).

9. The combination method of the multiple-layer conductive physiological detection structure as claimed in any of the preceding claims further comprising at least two pieces of conductive fabric (100), the conductive woven band (200) being positioned between the pieces of conductive fabric (100) to be combined therewith and connected thereto.

10. The combination method of the multiple-layer conductive physiological detection structure as claimed in Claim **3**, wherein the three-dimensional mixed-yarn conductive fabric (300) further comprises a protrusion member contained therein.

11. The combination method of the multiple-layer conductive physiological detection structure as claimed in Claim **10**, wherein the protrusion member (400) comprises foam, silicon rubber, or cotton.

12. The combination method of the multiple-layer conductive physiological detection structure as claimed in Claim **5**, wherein the yarns are formed of chemical fibers, natural fibers, or mix spinning of chemical fibers and natural fibers.

13. The combination method of the multiple-layer conductive physiological detection structure as claimed in any of the preceding claims, wherein the conductive woven band (200) comprises an electrically conductive unit (201), which is wrapped around or extends across the conductive woven band (200) in a nonlinear manner.

14. The combination method of the multiple-layer conductive physiological detection structure as claimed in Claim **5 or 7 or 8,** wherein the electrically conductive unit (201) comprises a conductive yarn or a conductive wire.

15. The combination method of the multiple-layer conductive physiological detection structure as claimed in any of the preceding claims, wherein the conductive woven band (200) further comprises at least two elastic yarns, the elastic yarns being arranged at two lateral sides of the conductive woven band (200).
